# EUROPEAN PATENT SPECIFICATION

(11) **EP 4 178 507 B1**
(45) Date of publication and mention of the grant of the patent: **29.01.2025**
(21) Application number: 21746564.0
(22) Date of filing: 02.07.2021
(51) Int. Cl.: A61F 9/007

(54) **OPHTHALMIC CURETTE**
OPHTHALMISCHE KÜRETTE
CURETTE OPHTALMIQUE

(30) Priority: 07.07.2020 US 202016922200
(43) Date of publication of application: 17.05.2023
(73) Proprietor: Johnson & Johnson Surgical Vision, Inc., Irvine, CA 92618 (US)
(72) Inventor: ALGAWI, Yehuda, 2066717 Yokneam (IL); GOVARI, Assaf, 2066717 Yokneam (IL); SITNITSKY, Ilya, 2066717 Yokneam (IL); ALTMANN, Andres Claudio, 2066717 Yokneam (IL); AHARON, Eran, 2066717 Yokneam (IL)
(74) Representative: Carpmaels & Ransford LLP
(86) International application number: PCT/IB2021/055963
(87) International publication number: WO 2022/009048

(56) References cited:
- DE-A1- 10 038 015
- US-A- 4 811 734
- US-B2- 10 687 978

## Description

### FIELD OF THE INVENTION

The present invention relates to medical devices, and in particular to, ophthalmic curettes.

### BACKGROUND

A cataract is a clouding and hardening of the eye's natural lens, a structure which is positioned behind the cornea, iris and pupil. The lens is mostly made up of water and protein and as people age these proteins change and may begin to clump together obscuring portions of the lens. To correct this a physician may recommend phacoemulsification cataract surgery. Before the procedure the surgeon numbs the area with anesthesia. Then a small incision is made in the cornea of the eye. Fluids are injected into this incision to support the surrounding structures. The anterior surface of the lens capsule is then removed to gain access to the cataract. The surgeon then uses a phacoemulsification probe, which has an ultrasonic handpiece with a titanium or steel needle. The tip of the needle vibrates at ultrasonic frequency to sculpt and emulsify the cataract, while a pump device aspirates particles from the cataract through the tip. The pump is typically controlled with a microprocessor. The pump may be a peristaltic or a venturi type of pump. Aspirated fluids are replaced with irrigation of a balanced salt solution to maintain the anterior chamber of the eye. After removing the cataract with phacoemulsification, the softer outer lens cortex is removed with suction. An intraocular lens (IOL) is introduced into the empty lens capsule. Small struts called haptics help hold the IOL in place. Once correctly implanted the IOL restores the patient's vision.

Chinese Patent 203122746 to Liu describes an ophthalmic curette, which includes a handle, a neck portion, a curette head, a light-emitting diode (LED) lamp, a button cell groove and a switch. The neck portion is connected with the handle, the curette head is connected with the neck portion, the LED lamp is mounted on the handle, and the button cell groove is connected with the LED lamp and the switch through wires.

Chinese Patent 202724100 to Tianjin Binhai New Area Dagang Hospital describes a curette used for ophthalmology and comprises a curette head, a curette neck, a curette handle, and an injector connector, wherein the curette head is a circular spoon-shaped deboss structure. The curette neck and the curette handle are both of a hollow tubular structure. An opening is arranged at the front end of the curette neck. The back end of the curette handle is provided with the injector connector, and the injector connector can be connected with an injector so as to conveniently inject irrigation liquid and medicine into a diseased portion. By means of the curette, the diseased position can be irrigated or injected with the medicine after diseased tissue is scraped off.

US Patent 10,238,417 to Carpenter describes a curette used in debridement and removal of skin, tissue, and necrotic debris from wound beds comprising a pinchable cap and a circular metal cutting end, wherein an individual may apply pressure to the cap, covering the metal cutting end, in order to manipulate the cutting end into a shape to debride narrow or smaller portions of a wounds such as a narrow oval. The curette also includes a measurement scale, a depth probe that also acts as a packing tool, and finger grips. An optional LED or LEDs may be included in or on the curette.

US 4,811,734 describes a surgical cutting instrument comprising an outer tube having a peripheral wall and a longitudinal axis. The outer tube has a plurality of openings arranged generally longitudinally along the outer tube. Each of the openings has first and second cutting edges defining portions of the periphery of such opening. Each of the first cutting edges is substantially straight as viewed in a particular direction perpendicular to the longitudinal axis of the outer tube. An inner cutting member to rotatable within the outer tube. The inner cutting member has a cutting edge cooperable with the first and second cutting edges of the openings of the outer tube for cutting material from within the patient with a shearing action.

### SUMMARY

There is provided in accordance with the present invention, an ophthalmic curette device according to independent claim 1.

Additionally, in accordance with an embodiment of the present disclosure the magnetic position sensor includes at least one coil.

Moreover, in accordance with an embodiment of the present disclosure the tube has a minimum length of 2 cm.

Further in accordance with an embodiment of the present disclosure the tube has an outer diameter between 0.4 mm and 0.8 mm.

Still further in accordance with an embodiment of the present disclosure the tube has a wall thickness between 0.03 mm and 0.2 mm.

Additionally, in accordance with an embodiment of the present disclosure, the device includes a loop or hook connected to the distal end of the tube.

Moreover, in accordance with an embodiment of the present disclosure, the device includes a processor configured to compute a pressure value responsively to the signal provided by the pressure sensor, and a display configured to render the pressure value.

Further in accordance with an embodiment of the present disclosure the processor is disposed in the handle, and the display is disposed on the handle.

There is also provided in accordance with another embodiment of the present disclosure, an ophthalmic curette device, including a handle having a distal end, a tube having a proximal end connected to the distal end of the handle, and having a distal end, and configured to be inserted through a cataract incision into a chamber of an eye of a living subject, and a temperature sensor disposed inside the tube at the distal end of the tube and configured to provide a signal responsively to a temperature inside the chamber of the eye.

Still further in accordance with an embodiment of the present disclosure, the device includes a magnetic position sensor disposed inside the tube.

Additionally, in accordance with an embodiment of the present disclosure the magnetic position sensor includes at least one coil.

Moreover, in accordance with an embodiment of the present disclosure the tube has a minimum length of 2 cm.

Further in accordance with an embodiment of the present disclosure the tube has an outer diameter between 0.4 mm and 0.8 mm.

Still further in accordance with an embodiment of the present disclosure the tube has a wall thickness between 0.03 mm and 0.2 mm.

Additionally, in accordance with an embodiment of the present disclosure, the device includes a loop or hook connected to the distal end of the tube.

Moreover, in accordance with an embodiment of the present disclosure, the device includes a processor configured to compute a temperature value responsively to the signal provided by the temperature sensor, and a display configured to render the temperature value.

Further in accordance with an embodiment of the present disclosure the processor is disposed in the handle, and the display is disposed on the handle.

### BRIEF DESCRIPTION OF THE DRAWINGS

The present invention will be understood from the following detailed description, taken in conjunction with the drawings in which:
Fig. 1 is a partly pictorial, partly block diagram view of an ophthalmic curette device constructed and operative in accordance with an embodiment of the present invention;
Fig. 2 is a schematic view of the device of Fig. 1 being inserted into an eye of a living subject;
Fig. 3 is a cross-sectional view of the device through line A: A of Fig. 1;
Figs. 4 and 5 are views of the distal end of the device of Fig. 1;
Fig. 6 is a cutaway view of the distal end of the device of Fig. 1; and
Fig. 7 is a schematic view of a distal end of an ophthalmic curette device constructed and operative in accordance with an alternative embodiment of the present invention.

### DESCRIPTION OF EXAMPLE EMBODIMENTS OVERVIEW

During cataract surgery, a phacoemulsification probe is typically used with an ophthalmic curette to assist in removal of the cataract from the eye. The curette and the phacoemulsification probe require separate incisions into the eye. However, to make measurements on the eye during the surgery, such as measuring intraocular pressure and/or temperature, different probes need to be used.

Embodiments of the present invention solve the above problems by providing an ophthalmic curette which includes a tube at its distal end for being inserted through a cataract incision into a chamber of an eye of a living subject. One or more sensors are placed inside the tube, typically at its distal end, in order to take pressure and/or temperature readings in the chamber. The ophthalmic curette may therefore be used to manipulate cataract material during a procedure as well as provide pressure and/or temperature readings thereby removing the need for further tools to be used and/or further incisions. In some embodiments, a position sensor may be placed in the tube, so that the distal end of the curette may be tracked using a position tracking system.

Some embodiments include an ophthalmic curette device, which includes a handle and a tube (for example, a polymer tube or a metal tube such as a rigid biocompatible metal tube) having a proximal end connected to a distal end of the handle. The distal end of the tube is inserted through a cataract incision into a chamber of an eye of a living subject. The tube may have any suitable cross-section shape for example, circular, elliptical, or rectangular. The tube may have any suitable form. In some embodiments, the tube is straight. In other embodiments, the tube is bent or curved. The tube may be formed from any suitable material, e.g., a polymer such as Polyether ether ketone (PEEK) or fluorinated ethylene propylene (FEP), or a metal such as stainless steel or titanium.

In some embodiments, the device also includes a pressure sensor placed inside the distal end of tube, and provides a signal responsively to intraocular pressure inside the chamber of the eye. In some embodiments, the device includes a temperature sensor placed inside the distal end of the tube and provides a signal responsively to a temperature inside the chamber of the eye.

In some embodiments, the device includes a magnetic position sensor placed inside the tube. The magnetic position sensor may be configured to track a position (e.g., location and/or orientation) of the distal end of the device. For example, magnetic field generators generating an alternating magnetic field may be placed around the neck and/or head of the living subject. The generated fields are then detected by the magnetic position sensor which provides a signal indicative of the position of the distal end with respect to the magnetic field generators. The magnetic position sensor may enable the device to be used as a tool during robotic guided surgery.

The tube may have any suitable length, which is long enough for the tube to be inserted into the eye and be maneuvered during the medical procedure. In some embodiments, the tube has a minimum length of 2 cm. The tube may have any suitable outer diameter and wall thickness. In some embodiments, the tube has an outer diameter between 0.4 mm and 0.8 mm, and a wall thickness between 0.03 mm and 0.2 mm.

The device optionally includes a processor and a display. In some embodiments, the processor is configured to compute a pressure value responsively to the signal provided by the pressure sensor. In some embodiments, the processor is configured to compute a temperature value responsively to the signal provided by the temperature sensor. The display is configured to render the pressure value and/or the temperature value. In an embodiment, the processor and the display may be disposed in the handle. In some embodiments, a display may also be disposed in a remote console, which is connected to the handle wirelessly and/or via wires, to also render the pressure value and/or the temperature value. The handle and the console may each include an interface to provide data communication between the handle and the console. In an embodiment, a processor may be disposed in the handle or the console. In other embodiments, the display and/or the processor are not included in the handle or the console.

In some embodiments, the sensor signal(s) and/or computed temperature and/or pressure values are conveyed via wires and/or wirelessly to a remote processing device which processes the sensor signals and/or uses the computed temperature and/or pressure values as part of a medical procedure process. For example, medical procedure parameters such as phacoemulsification probe needle vibration frequency, amplitude, and/or mode may be adjusted according to the temperature and/or pressure values.

### SYSTEM DESCRIPTION

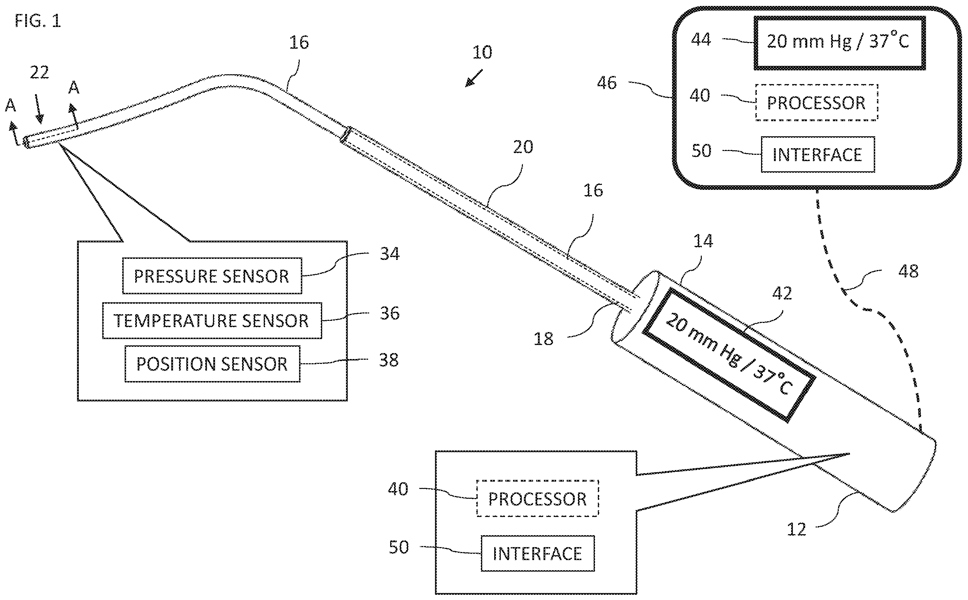

Reference is now made to Fig. 1, which is a partly pictorial, partly block diagram view of an ophthalmic curette device 10 constructed and operative in accordance with an embodiment of the present invention.

The ophthalmic curette device 10 includes a handle 12 having a distal end 14. The handle 12 may be formed from any suitable material or combination of materials, for example, a metal and/or polymer.

The ophthalmic curette device 10 includes a tube 16 having a proximal end 18 connected to the distal end 14 of the handle 12. The tube 16 may have any suitable form. In some embodiments, the tube 16 is straight. In other embodiments, the tube 16 is bent or curved to any suitable angle, as shown in Fig. 1. The tube 16 may be formed from any suitable metal, for example, stainless steel or titanium. In some embodiments, the tube 16 may be replaced by a tube of any suitable material, for example, a polymer such as PEEK or FEP. Suitable metal and polymer tubing are commercially available from IDEX corporation, Lake Forest, Illinois, USA.

A proximal portion of the tube 16 is optionally surrounded by an elongated supporting member 20 to provide additional support to the proximal portion which is not inserted into an eye. The supporting member 20 may be formed from any suitable material, for example, a metal such as stainless steel or titanium, or a polymer. The tube 16 has a distal end 22.

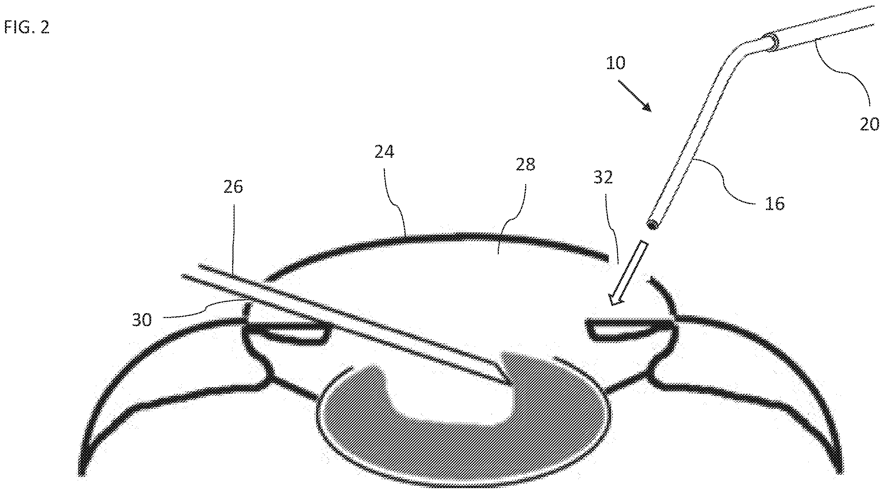

Reference is now made to Fig. 2, which is a schematic view of the device 10 of Fig. 1 being inserted into an eye 24 of a living subject.

Fig. 2 shows a needle 26 of a phacoemulsification probe already inserted into a chamber 28 of the eye 24 through an incision 30. The tube 16 of the ophthalmic curette device 10 is configured to be inserted through a second incision 32 into the chamber 28 of the eye 24.

The tube 16 (which extends beyond the supporting member 20) may have any suitable length, which is long enough for the tube 16 to be inserted into the eye 24 and be maneuvered during the medical procedure. In some embodiments, the tube 16 (which extends beyond the supporting member 20) has a minimum length of 2 cm. The tube 16 may have any suitable outer diameter and wall thickness. In some embodiments, the tube 16 has an outer diameter between 0.4 mm and 0.8 mm, and a wall thickness between 0.03 mm and 0.2 mm.

Referring again to Fig. 1., in some embodiments, the ophthalmic curette device 10 includes a pressure sensor 34 disposed inside the tube 16 at the distal end 22 of the tube 16. The pressure sensor 34 is configured to provide a signal responsively to intraocular pressure inside the chamber 28 (Fig. 2) of the eye 24 (Fig. 2). Any suitable pressure sensor, which is small enough to insert inside the tube 16, and is sensitive enough to accurately measure the intraocular pressure, may be used. A suitable pressure sensor (e.g., P330B) is commercially available from Amphenol Thermometrics Inc., St. Mary's, PA 15857, United States. The P330B pressure sensor has high pressure sensitivity and has a size of 330 x 900 x 120 microns. The longest dimension of the P330B pressure sensor may be aligned parallel to the axis of the tube 16.

In some embodiments, the ophthalmic curette device 10 includes a temperature sensor 36 disposed inside the tube 16 at the distal end 22 of the tube 16. The temperature sensor 36 is configured to provide a signal responsively to a temperature inside the chamber 28 (Fig. 2) of the eye 24 (Fig. 2). Suitable miniature medical temperature sensors are commercially available from ATC Semitec Ltd, Anderton, Northwich, Cheshire, CW9 6FY, U.K.

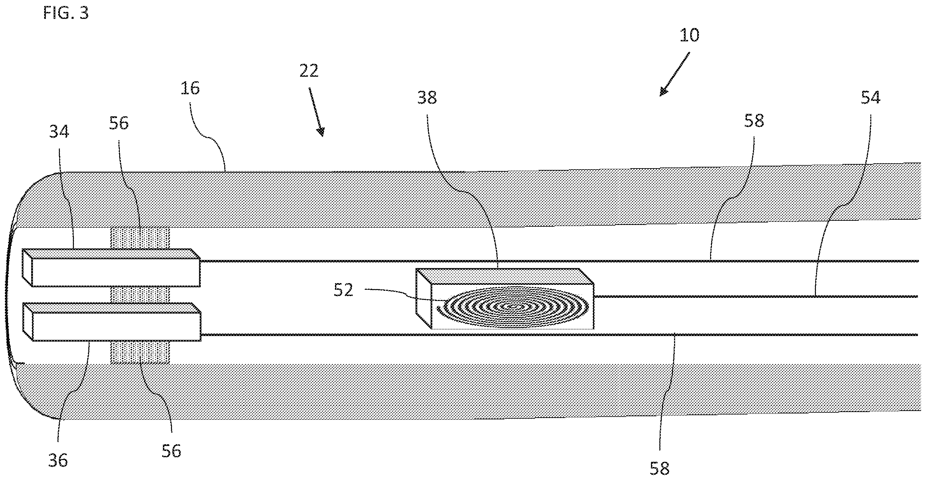

In some embodiments, the ophthalmic curette device 10 includes the pressure sensor 34 and the temperature sensor 36. The pressure sensor 34 and the temperature sensor 36 are described in more detail with reference to Fig. 3.

In some embodiments, the ophthalmic curette device 10 includes a magnetic position sensor 38 disposed inside the tube 16. The magnetic position sensor 38 may be configured to be used as part of a position tracking system (not shown) for tracking a position of the distal end 22 of the tube 16. As the tube 16 is rigid, the magnetic position sensor 38 may be disposed at any suitable position inside the tube 16. The magnetic position sensor 38 is described in more detail with reference to Fig. 3.

The ophthalmic curette device 10 optionally includes a processor 40 and a display 42. In some embodiments, the processor 40 is configured to compute a pressure value responsively to the signal provided by the pressure sensor 34. In some embodiments, the processor 40 is configured to compute a temperature value responsively to the signal provided by the temperature sensor 36. The display 42 is configured to render the pressure value and/or the temperature value.

The processor 40 and the display 42 may be disposed in/on the handle 12. The display 42 may include any suitable display, for example, a liquid crystal display.

In some embodiments, a display 44 may also be disposed in a remote console 46, which is connected with the handle 12 wirelessly and/or via wires 48. The display 44 is configured to render the pressure value and/or the temperature value. The handle 12 and the console 46 may each include an interface 50 to provide data communication between the handle 12 and the console 46. The interfaces 50 may comprise circuitry to allow wired and/or wireless communication therebetween.

In some embodiments, the display 44 may be disposed in the remote console 46 without a display on the handle 12. In these embodiments, the processor 40 may be disposed in the handle 12 or the console 46.

In practice, some or all of the functions of the processor 40 may be combined in a single physical component or, alternatively, implemented using multiple physical components. These physical components may comprise hard-wired or programmable devices, or a combination of the two. In some embodiments, at least some of the functions of the processor 40 may be carried out by a programmable processor under the control of suitable software. This software may be downloaded to a device in electronic form, over a network, for example. Alternatively, or additionally, the software may be stored in tangible, non-transitory computer-readable storage media, such as optical, magnetic, or electronic memory.

In other embodiments, the displays 42, 44 and/or the processor 40 are not included in the handle 12 or the console 46.

In some embodiments, the sensor signal(s) and/or computed temperature and/or pressure values are conveyed via wires and/or wirelessly to a remote processing device (not shown) which processes the sensor signals and/or uses the computed temperature and/or pressure values as part of a medical procedure process. For example, medical procedure parameters such as phacoemulsification probe needle vibration frequency, amplitude, and/or mode may be adjusted according to the temperature and/or pressure values.

Reference is now made to Fig. 3, which is a cross-sectional view of the ophthalmic curette device 10 through line A:A of Fig. 1.

The magnetic position sensor 38 may include at least one coil 52. The magnetic position sensor 38 may be implemented as a single axis sensor (SAS) with one coil, as a dual axis sensor (DAS) with two orthogonally disposed coils, or a triple axis sensor (TAS) with three orthogonally disposed coils. Each coil may be a printed coil or a wound coil, e.g., wound on a suitable magnetic core. The ophthalmic curette device 10 may include a cable 54 connecting the magnetic position sensor 38 with the processor 40 (Fig. 1) or the interface 50 (Fig. 1) in the handle 12 (Fig. 1).

Magnetic field generators may be placed at known locations external to the patient, for example, around the head of the patient or in a collar around the neck of the patient. The magnetic position sensor 38 generates electrical signals in response to these magnetic fields, which may be processed to determine a position (e.g., location and/or orientation) of the distal end 22 of the ophthalmic curette device 10. Magnetic tracking system are described in U.S. Pat. Nos. 5,391,199, 6,690,963, 6,484,118, 6,239,724, 6,618,612 and 6,332,089, in PCT International Publication No. WO 1996/005768, and in U.S. Patent Application Publications Nos. 2002/006455 and 2003/0120150 and 2004/0068178. The magnetic position sensor 38 may allow the ophthalmic curette device 10 to be tracked during use, for example, during robotic guided surgery in which the ophthalmic curette device 10 may be robotically guided.

In some embodiments, the coil may include a magnetic core. For example, magnetic antennas can use a magnetic core made of a ferromagnetic or ferrimagnetic material such as iron or a nickel-zinc ferrite or magnesium-zinc ferrite to increase permeability. A magnetic core can increase the sensitivity of an antenna by a factor of up to several thousand, by increasing the magnetic field due to its higher magnetic permeability. Therefore, coils used in navigable probes typically include coils with a magnetic core. A solid magnetic core may be constructed by any suitable method including joining magnetic-core powder using a binder and/or very high temperatures (sintering) to form a solid mass. In some embodiments, the coil may include a magnetic core produced by one of the above methods. However, the above production methods are generally not suitable for producing solid magnetic cores which are small enough to insert into a coil having an inner diameter of about 500 microns or less.

Therefore, in some embodiments, a magnetic core may be formed from a tube containing separate powder granules of a ferrite. The tube may have any suitable inner diameter. The inner diameter of the tube may be in the range of 100 to 750 microns. A coil is then placed around the tube, for example, by inserting the tube into the coil. The coil may be covered with a covering keeping the coil in place and acting as a biocompatible cover, for example, but not limited to a plastic cover such as a plastic tube, or with a coating such an as enamel or epoxy paint, shrink sleeve, or metal cover. A metal cover may also provide shielding from high frequency electromagnetic interference. The wire (e.g., copper wire) used in the coil may have any suitable gauge, for example, but not limited to 60 gauge which is about 8 microns in diameter. The powder granules are held in place by the tube. The powder granules may be bound together using a binder material such as epoxy. The tube may be formed from any suitable material such as a wide-range of thermoplastics, e.g., polyimide, polyamide, polyethylene terephthalate (PET), fluorinated ethylene propylene (FEP), or polyvinyl chloride (PVC) or other materials such as an engineered ceramic, a carbon material, or a non-ferromagnetic metal. The tube provides a controlled outer diameter surface on which to slide the coil.

As the powder granules may have a size of about 40 microns, it may be difficult to place the powder granules into the tube. Therefore, in some embodiments, separate powder granules of a ferrite are introduced into the tube using the following method. The tube may be made of any suitable material which can be heat-shrunk, for example, but not limited to, a wide-range of thermoplastics, e.g., polyamide, polyethylene terephthalate (PET), fluorinated ethylene propylene (FEP), or polyvinyl chloride (PVC). While the powder granules are introduced into the tube, the tube typically has an outer diameter which is greater than an inner diameter of the coil. For example, if the inner diameter of the coil is 180 microns, the outer diameter of the tube in to which the powder granules are disposed has an outer diameter of 250 microns and an inner diameter of about 180 microns. The outer diameter of the tube shrinks to 180 microns after being heat shrunk. The preshrunk tube may have any suitable outer diameter in accordance with the inner diameter of the coil and the heat shrink properties of the plastic tube. The preshrunk tube may have any suitable outer diameter, for example, in the range of 150 - 1200 microns. The powder granules may be bound together after being placed in the tube using a binder such as a low viscosity epoxy, which may be wicked in to the tube with capillary action after the tube is shrunk, or before the tube is shrunk and then the tube is shrunk while the epoxy is still liquid as it would not be possible to shrink the tube once the epoxy is cured. The powder granules may have any suitable size which is less than the inner diameter of the tube in to which the powder granules are disposed. The powder granules may be introduced into the tube using any suitable method. For example, the powder granules may be introduced into the tube with the aid of a funnel which is aligned with the tube opening. The funnel is filled with the powder granules and is vibrated up and down, and/or side-to-side, using any suitable vibration method, such as an ultrasonic method. The funnel may be connected with the tube, and vibrated in unison with the tube to facilitate the introduction of the powder granules into the tube. Additionally, or alternatively, one or more magnets (electromagnetics and/or permanent magnets) may be connected to the tube to facilitate introduction of the powder granules into the tube. For example, using a magnet connected to the bottom of the tube and/or a ring-type magnet connected around the tube. The magnet(s) may allow movement (up and down, and/or sideways movement), vibration (up and down, and/or sideways vibration), and/or rotation of the tube.

In some embodiments, the powder granules may be introduced into a tube without subsequently heat shrinking the tube. In these embodiments, the powder granules are suspended in a liquid, such as an alcohol, for example, but not limited to, isopropyl alcohol, or any other liquid which has a sufficiently low viscosity and a high enough evaporation rate. The suspending may be performed using any suitable method for example, but not limited to, placing the powder granules with the liquid in a container on a vibration table or by using any other vibration method, such as using ultrasound. The percentage of powder granules in the suspension, by volume, may be any suitable value, for example, in the range of 30-70%. An end of an empty tube is placed in the liquid so that capillary action draws some of the liquid with the powder granules into the tube. The tube has an outer diameter less than the inner diameter of the coil, for example, in the range of 150 to 800 microns. The inner diameter of the tube is generally in the range of 100 to 750 microns. The tube may be made of any suitable material, for example, but not limited to, plastic, an engineered ceramic, a carbon material, or a non-ferromagnetic metal. Once the liquid-granule mixture is in the tube, the liquid is evaporated from the tube using any suitable method such as using heat and/or by blowing air over the tube. After the evaporation, a binder such as a low viscosity epoxy may be wicked into the tube with capillary action to bind the powder granules together.

The pressure sensor 34 may be disposed at the distal tip of the tube 16. In some embodiments, the temperature sensor 36 may be disposed next to the pressure sensor 34 at the distal tip if there is enough room in the distal tip for both sensors 34, 36. The pressure sensor 34 and the temperature sensor 36 may be connected to the inner surface of the tube 16 using a suitable adhesive 56, which also prevents liquid from reaching electrical connections, wires and the magnetic position sensor 38.

If there is not enough room for both sensors 34, 36 at the distal tip, the pressure sensor 34 is generally disposed at the distal tip and the temperature sensor 36 is disposed proximally to the pressure sensor 34, or vice-versa. The magnetic position sensor 38 is generally, but not necessarily, disposed proximally to the pressure sensor 34 and the temperature sensor 36.

The pressure sensor 34 and the temperature sensor 36 may be connected using wires/cables 58 to the processor 40 (Fig. 1) and/or the interface 50 (Fig. 1) in the handle 12 (Fig. 1). In some embodiments, the wires/cables 58 may extend out of the handle 12 for connection to the remote console 46 (Fig. 1).

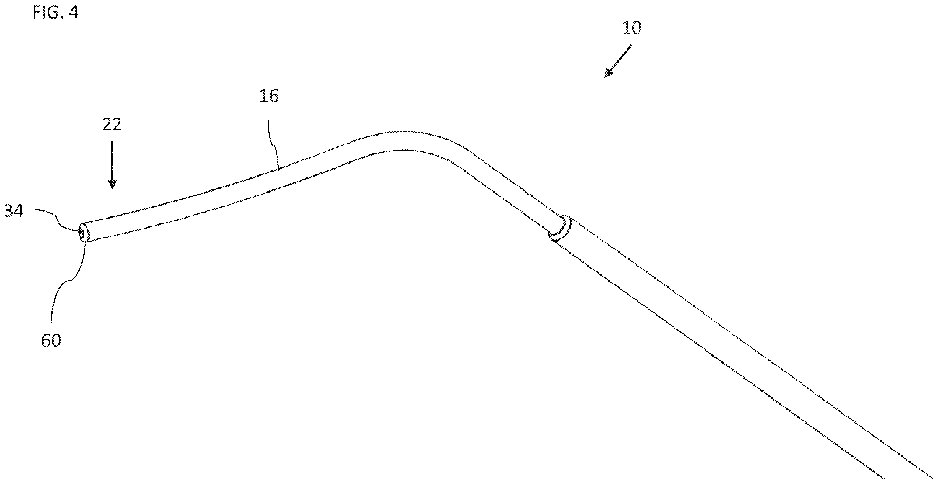

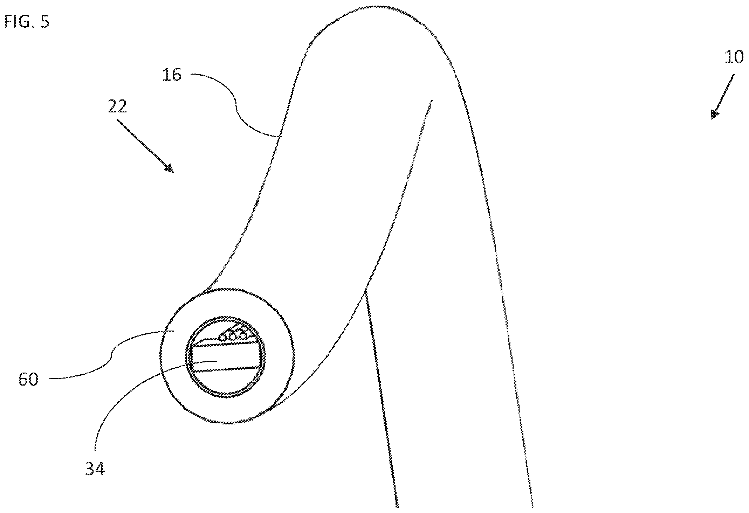

Reference is now made to Figs. 4 and 5, which are views of the distal end 22 of the ophthalmic curette device 10 of Fig. 1. Fig. 4 shows that the distal end 22 has a smooth or rounded edge 60. Fig. 5 shows the pressure sensor 34 disposed in the distal end 22 of the tube 16. The temperature sensor 36 and the magnetic position sensor 38 are not shown in Figs. 4 and 5 for the sake of simplicity.

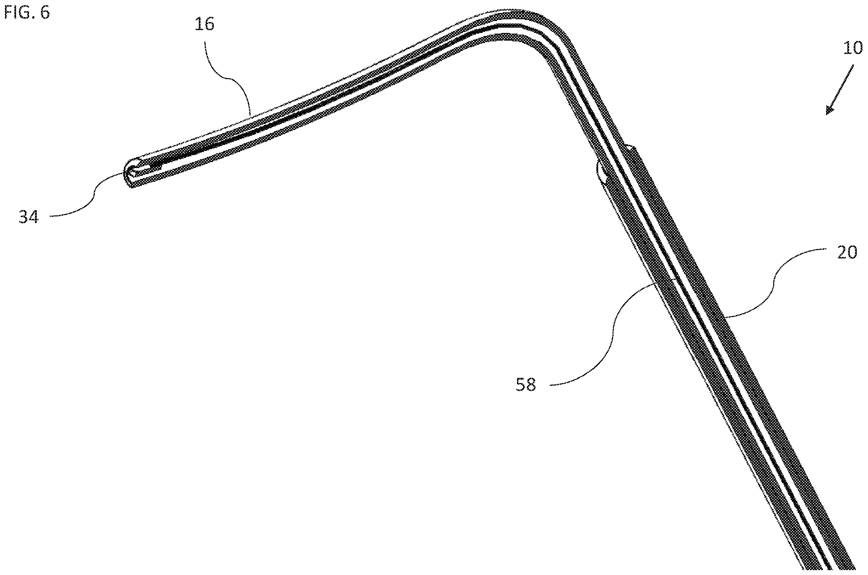

Reference is now made to Fig. 6, which is a cutaway view of the distal end of the ophthalmic curette device 10 of Fig. 1. Fig. 6 shows the cables 58 extending from the pressure sensor 34 down the inside of the tube 16 towards the handle 12 (Fig. 1). Fig. 6 also illustrates the supporting member 20 surrounding the proximal portion of the tube 16. The temperature sensor 36, the magnetic position sensor 38, and their associated wires/cables are not shown in Fig. 6 for the sake of simplicity.

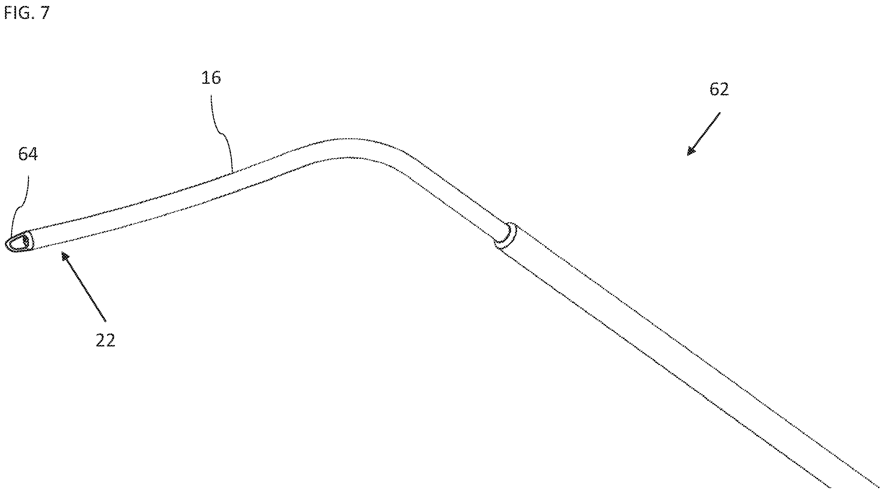

Reference is now made to Fig. 7, which is a schematic view of a distal end of an ophthalmic curette device 62 constructed and operative in accordance with an alternative embodiment of the present invention. The ophthalmic curette device 62 is substantially the same as the ophthalmic curette device 10 except that the ophthalmic curette device 62 includes a loop 64 or hook connected to the distal end 22 of the tube 16. The width of the loop is typically about the size of the outer diameter of the tube 16. The length of the loop 64 extending away from the distal end 22 may have any suitable size, for example, between half and twice the size of the outer diameter of the tube 16.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 72% to 108%.

Various features of the invention which are, for clarity, described in the contexts of separate embodiments may also be provided in combination in a single embodiment. Conversely, various features of the invention which are, for brevity, described in the context of a single embodiment may also be provided separately or in any suitable sub-combination.

The embodiments described above are cited by way of example, and the present invention is not limited by what has been particularly shown and described hereinabove. Rather the scope of the invention includes both combinations and subcombinations of the various features described hereinabove, as well as variations and modifications thereof which would occur to persons skilled in the art upon reading the foregoing description and which are not disclosed in the prior art.

## Claims

1. An ophthalmic curette device (10), comprising:
a handle (14) having a distal end;
a tube (16) having a proximal end (18) connected to the distal end of the handle, and having a distal end (22), and configured to be inserted through an incision into an eye;
**characterized in** the device comprises
a magnetic position sensor (38) disposed inside the tube; and further comprises one or both of:
a pressure sensor (34) disposed inside the tube at the distal end of the tube, and configured to provide a signal responsively to intraocular pressure inside the eye; and
a temperature sensor (36) disposed inside the tube at the distal end of the tube and configured to provide a signal responsively to a temperature inside the eye.

2. The device according to claim 1, wherein the tube is a metal tube.

3. The device according to claim 1, wherein the tube is a polymer tube.

4. The device according to claim 1, wherein the magnetic position sensor includes at least one coil (52).

5. The device according to claim 1, wherein the tube has a minimum length of 2 cm.

6. The device according to claim 5, wherein the tube has an outer diameter between 0.4 mm and 0.8 mm.

7. The device according to claim 6, wherein the tube has a wall thickness between 0.03 mm and 0.2 mm.

8. The device according to claim 1, further comprising a loop or hook (64) connected to the distal end of the tube.

9. The device according to claim 1, wherein the device comprises the pressure sensor, the device further comprising:
a processor (40) configured to compute a pressure value responsively to the signal provided by the pressure sensor; and
a display (44) configured to render the pressure value.

10. The device according to claim 1, wherein the device comprises the temperature sensor, the device further comprising:
a processor (40) configured to compute a temperature value responsively to the signal provided by the temperature sensor; and
a display (44) configured to render the temperature value.

11. The device according to claim 9 or claim 10, wherein:
the processor is disposed in the handle; and
the display is disposed on the handle.

## Patentansprüche

1. Augenkürettenvorrichtung (10), umfassend:
einen Griff (14), der ein distales Ende aufweist;
ein Rohr (16), das ein proximales Ende (18) aufweist, das mit dem distalen Ende des Griffs verbunden ist, und ein distales Ende (22) aufweist und konfiguriert ist, um durch einen Einschnitt in ein Auge eingeführt zu werden;
**dadurch gekennzeichnet, dass** die Vorrichtung umfasst
einen magnetischen Positionssensor (38), der innerhalb des Rohrs angeordnet ist; und ferner eines oder beides umfasst von:
einen Drucksensor (34), der im Inneren des Rohrs an dem distalen Ende des Rohrs angeordnet und konfiguriert ist, um als Reaktion auf einen intraokularen Druck im Inneren des Auges ein Signal bereitzustellen; und
einen Temperatursensor (36), der im Inneren des Rohrs an dem distalen Ende des Rohrs angeordnet und konfiguriert ist, um als Reaktion auf eine Temperatur im Inneren des Auges ein Signal bereitzustellen.

2. Vorrichtung nach Anspruch 1, wobei das Rohr ein Metallrohr ist.

3. Vorrichtung nach Anspruch 1, wobei das Rohr ein Polymerrohr ist.

4. Vorrichtung nach Anspruch 1, wobei der magnetische Positionssensor mindestens eine Spule (52) einschließt.

5. Vorrichtung nach Anspruch 1, wobei das Rohr eine Mindestlänge von 2 cm aufweist.

6. Vorrichtung nach Anspruch 5, wobei das Rohr einen Außendurchmesser zwischen 0,4 mm und 0,8 mm aufweist.

7. Vorrichtung nach Anspruch 6, wobei das Rohr eine Wanddicke zwischen 0,03 mm und 0,2 mm aufweist.

8. Vorrichtung nach Anspruch 1, ferner umfassend eine Schlaufe oder einen Haken (64), die/der mit dem distalen Ende des Rohrs verbunden ist.

9. Vorrichtung nach Anspruch 1, wobei die Vorrichtung den Drucksensor umfasst, die Vorrichtung ferner umfassend:
einen Prozessor (40), der konfiguriert ist, um als Reaktion auf das Signal, das durch den Drucksensor bereitgestellt wird, einen Druckwert zu berechnen; und
eine Anzeige (44), die konfiguriert ist, um den Druckwert wiederzugeben.

10. Vorrichtung nach Anspruch 1, wobei die Vorrichtung den Temperatursensor umfasst, die Vorrichtung ferner umfassend:
einen Prozessor (40), der konfiguriert ist, um als Reaktion auf das Signal, das durch den Temperatursensor bereitgestellt wird, einen Temperaturwert zu berechnen; und
eine Anzeige (44), die konfiguriert ist, um den Temperaturwert wiederzugeben.

11. Vorrichtung nach Anspruch 9 oder 10, wobei:
der Prozessor in dem Griff angeordnet ist; und
die Anzeige an dem Griff angeordnet ist.

## Revendications

1. Dispositif de curette ophtalmique (10), comprenant :
une poignée (14) ayant une extrémité distale ;
un tube (16) ayant une extrémité proximale (18) reliée à l'extrémité distale de la poignée, et ayant une extrémité distale (22), et conçu pour être inséré à travers une incision dans un oeil ;
**caractérisé en ce que** le dispositif comprend
un capteur de position magnétique (38) disposé à l'intérieur du tube ; et comprend en outre un ou deux éléments parmi :
un capteur de pression (34) disposé à l'intérieur du tube à l'extrémité distale du tube, et configuré pour fournir un signal en réponse à la pression intraoculaire à l'intérieur de l'œil ; et
un capteur de température (36) disposé à l'intérieur du tube à l'extrémité distale du tube et configuré pour fournir un signal en réponse à une température à l'intérieur de l'œil.

2. Dispositif selon la revendication 1, dans lequel le tube est un tube métallique.

3. Dispositif selon la revendication 1, dans lequel le tube est un tube en polymère.

4. Dispositif selon la revendication 1, dans lequel le capteur de position magnétique comporte au moins une bobine (52).

5. Dispositif selon la revendication 1, dans lequel le tube a une longueur minimale de 2 cm.

6. Dispositif selon la revendication 5, dans lequel le tube a un diamètre extérieur compris entre 0,4 mm et 0,8 mm.

7. Dispositif selon la revendication 6, dans lequel le tube a une épaisseur de paroi comprise entre 0,03 mm et 0,2 mm.

8. Dispositif selon la revendication 1, comprenant en outre une boucle ou un crochet (64) relié à l'extrémité distale du tube.

9. Dispositif selon la revendication 1, dans lequel le dispositif comprend le capteur de pression, le dispositif comprenant en outre :
un processeur (40) configuré pour calculer une valeur de pression en fonction du signal fourni par le capteur de pression ; et
un dispositif d'affichage (44) configuré pour afficher la valeur de la pression.

10. Dispositif selon la revendication 1, dans lequel le dispositif comprend le capteur de température, le dispositif comprenant en outre :
un processeur (40) configuré pour calculer une valeur de température en fonction du signal fourni par le capteur de température ; et
un dispositif d'affichage (44) configuré pour afficher la valeur de la température.

11. Dispositif selon la revendication 9 ou la revendication 10, dans lequel :
le processeur est disposé dans la poignée ; et
le dispositif d'affichage est disposé sur la poignée.
